# EUROPÄISCHE PATENTSCHRIFT

(11) **EP 2 601 986 B1**
(45) Veröffentlichungstag und Bekanntmachung des Hinweises auf die Patenterteilung: **22.10.2014**
(21) Anmeldenummer: 12007466.1
(22) Anmeldetag: 02.11.2012
(51) Int. Cl.: A61M 3/02, A61B 17/16

(54) **Adapter für Bohrantrieb und Lavage-System**
Adapter for drill drive and lavage system
Adaptateur pour entraînement pour forage et système de lavage

(30) Priorität: 05.12.2011 DE 102011120087; 12.01.2012 DE 102012000392
(43) Veröffentlichungstag der Anmeldung: 12.06.2013
(73) Patentinhaber: Heraeus Medical GmbH, 61273 Wehrheim (DE)
(72) Erfinder: Vogt, Sebastian, Dr., 99092 Erfurt (DE); Apitius, Maria Katharina, 53424 Remagen (DE)
(74) Vertreter: Schultheiss & Sterzel Patentanwälte PartG mbB

(56) Entgegenhaltungen:
- WO-A1-96/25188
- DE-A1- 19 711 675
- DE-A1-102009 021 421
- US-A- 5 779 702
- US-A1- 2004 087 958

## Beschreibung

Die Erfindung betrifft einen Adapter für einen Bohrantrieb und ein Lavage-System sowie einen Bohrantrieb und ein Lavage-System mit einem Adapter.

Lavage-Systeme dienen dazu, bei einer Operation am Knochen, wie zum Beispiel dem Einsetzen eines künstlichen Hüftgelenks, Rückstände, die bei der Operation entstehen, schonend aber wirksam auszuspülen, um Komplikationen nach der Operation zu vermeiden und die Heilung zu beschleunigen. Die Rückstände können die Haltbarkeit eines künstlichen Hüftgelenks beeinträchtigen und zu Infektionen im Bereich des Knochens führen.

Dazu wird durch das Lavage-System ein pulsierender Strahl einer Flüssigkeit erzeugt. Die Flüssigkeit ist meist steriles Wasser beziehungsweise eine wässrige Lösung, die auch pharmazeutisch wirksame Substanzen enthält. Das Spülwasser mit den Rückständen wird durch das Lavage-System über einen zweiten Kanal abgesaugt.

Lavage-Systeme (auch Jet-Lavages genannt) umfassen eine Membranpumpe, die zum Erzeugen des pulsierenden Flüssigkeitsstrahls und zum Fördern der Flüssigkeit verwendet werden. Ein Antriebsmodul wird vor der Anwendung mit dem Lavage-System verbunden und dient dazu, die Membranpumpe anzutreiben.

Aus der nicht vorveröffentlichten DE 10 2010 046 057 B3 ist ein Lavage-System bekannt, bei dem einen linear oszillierenden Stößel durch einen Druckgasmotor angetrieben wird. Der Stößel schlägt periodisch auf eine Membran des Lavage-Systems und bildet dadurch eine periodisch arbeitende Membran-Pumpe zum Erzeugen des pulsierenden Flüssigkeitsstrahls.

Nachteilig ist hieran, dass der Motor mit Druckluft oder einem komprimierten Gas versorgt werden muss. Dazu muss der Druckgasmotor entweder an eine Druckgasleitung angeschlossen sein oder einen Kompressor mit elektrischen Leitungen oder eine Druckgaspatrone umfassen.

Die US 2003 036 723 A1 offenbart ein Lavage-System mit einer Pumpe, die mit einem Elektromotor über ein Getriebe angetrieben wird. Die Pumpe erzeugt den Flüssigkeitsstrahl. Die Energie für den Motor wird über ein Stromkabel zugeführt,

Nachteilig ist hieran der komplexe Aufbau des Lavage-Systems. Aufgrund der hygienischen Anforderungen müssen zumindest Teile des Lavage-Systems sterilisierbar sein oder das Lavage-System muss ein Wegwerfprodukt sein. Dies ist bei dem vorliegenden Lavage-System nach der US 2003 036 723 A1 teuer oder aufwendig.

Aus der US 5,779,702 A ist ein Adapter für ein Lavage-System bekannt, der auf einem Bohrer aufgesetzt werden kann. Dabei wird die Drehbewegung in eine lineare Bewegung eines Stößels umgesetzt. Die WO 96/25188 A1 offenbart eine Lavage-Pumpe, die über eine Exzenterscheibe angetrieben wird. Eine Rotationspumpe für ein chirurgisches Handinstrument wird in der DE 197 11 675 A1 beschrieben. Die US 2004/0087958 A1 offenbart einen biegbaren Antrieb für chirurgische Instrumente.

Die DE 10 2009 021 421 A1 schlägt eine Jet-Lavage mit einem Hubmagneten vor, der die Membranpumpe antreibt. Der Einmal-Artikel ist schon wesentlich kostengünstiger aufgebaut.

Nachteilig bleibt hieran, dass auch die Verwendung eines Hubmagneten Kosten verursacht und auch Ressourcen verbraucht. Die für den Aufbau von Magneten erforderlichen Elemente sind in letzter Zeit zunehmend teuer geworden. Beim Aufbau von Vorrichtungen mit Magneten sind magnetisierbare Materialien der Fertigungsteile zu vermeiden. Der Aufbau bleibt also immer noch aufwendig und kostenintensiv.

Aufgabe der Erfindung ist es, diese und weitere nicht genannte Nachteile zu überwinden. Insbesondere soll ein kostengünstigeres System bereitgestellt werden, das einfach eingesetzt und ressourcenschonend aufgebaut werden kann. Bevorzugt sollen bestehende Lavage-Systeme nutzbar bleiben.

Diese Aufgaben werden gelöst durch einen Adapter für einen Bohrantrieb zum Antreiben eines Lavage-Systems, umfassend eine erste Befestigungseinrichtung zum Anschließen des Adapters an den Bohrantrieb, eine zweite Befestigungseinrichtung zum Anschließen des Lavage-Systems an den Adapter und eine achsial drehbar gelagerte Welle, die an einem ersten Ende der Welle an den Bohrantrieb derart anschließbar ist, dass die Welle durch den Bohrantrieb drehbar ist, wobei der Adapter eine Scheibe umfasst, die an einem zweiten Ende der Welle gegenüber dem ersten Ende mit der Welle derart verbunden ist, dass sich bei einer Drehung der Welle auch die Scheibe dreht, wobei die Scheibe einen Aufsatz oder einen Vorsprung auf der von der Welle abgewandten Seite umfasst, der exzentrisch angeordnet ist, und/oder die Scheibe zur Drehachse der Welle geneigt ist, wobei der Adapter über die zweite Befestigungseinrichtung derart an das Lavage-System anschließbar ist, dass die drehende Scheibe auf der von der Welle abgewandten Seite oder über den Aufsatz oder den Vorsprung das Lavage-System antreibt, wobei der Aufsatz oder der Vorsprung oder die drehende Scheibe auf der von der Welle abgewandten Seite über einen Druckpunkt direkt auf eine Membran des Lavage-Systems drückt, wobei beim Drehen der Scheibe der Druckpunkt periodisch über die Membran läuft..

Die Welle kann im Sinne der vorliegenden Erfindung die erste Befestigungseinrichtung umfassen. Dazu kann ein Teil der Welle, der als erste Befestigungseinrichtung dient beispielsweise in ein Bohrfutter eines Bohrantriebs aufgenommen werden. Das angezogene Bohrfutter bildet mit dem aufgenommenen Teil der Welle eine Befestigung, die den Adapter mit dem Bohrantrieb verbindet und gleichzeitig für den Antrieb der Welle sorgt. Die Welle kann dazu erfindungsgemäß einen Stift umfassen und/oder eine oder mehrere Abflachungen aufweisen, die einen besseren Halt mit dem Bohrantrieb bieten.

Der Begriff Scheibe ist im Sinne der vorliegenden Erfindung breit auszulegen. Eine Scheibe kann erfindungsgemäß im Extremfall auch durch eine Stange mit im Wesentlichen zylinderförmigem Querschnitt ausgebildet sein. Besonders bevorzugt zur Ausgestaltung der vorliegenden Erfindung werden aber kreisförmige Scheiben, da diese sich ohne große Unwucht drehen lassen.

Dieser Aufbau ist besonders einfach und kostengünstig zu realisieren, da nur ein bewegliches Bauteil (Welle mit Scheibe und Aufsatz) in den Adapter eingebaut werden muss.

Alternativ dazu kann vorgesehen sein, dass an dem Aufsatz oder dem Vorsprung oder der Scheibe ein Pleuel über eine, um wenigstens eine Achse drehbare Verbindung an einem ersten Ende des Pleuels verbunden ist, so dass beim Drehen der Scheibe ein zweites Ende des Pleuels eine periodische Stoßbewegung vollführt, wobei vorzugsweise bei angeschlossenem Lavage-System das zweite Ende des Pleuels das Lavage-System antreibt, wobei besonders bevorzugt bei angeschlossenem Lavage-System das zweite Ende des Pleuels periodisch auf eine Membran des Lavage-Systems stößt.

Ein solcher Adapter ist zwar aufwendiger im Aufbau, bietet jedoch eine verbesserte Pumpleistung durch den Pleuel.

Eine weitere alternative Ausgestaltung kann vorsehen, dass der Adapter einen Kolben umfasst, der entlang der Kolbenachse beweglich und federnd gelagert ist, wobei der Kolben durch die drehende Scheibe periodisch in Richtung der Kolbenachse auslenkbar ist und durch die federnde Lagerung rückstellbar ist, wobei vorzugsweise die federnde Lagerung eine Feder, insbesondere eine Stahlfeder ist.

Dieser Adapter erfordert eine Federung und zwei bewegliche Bauteile, kann aber auf Gelenke verzichten. Durch den noch einfachen Aufbau und die gute Pumpleistung durch Verwendung eines linear beweglichen Kolbens sind solche Adapter besonders bevorzugt.

Bei dieser Ausführungsform kann vorgesehen sein, dass der Kolben auf der Seite zur Scheibe hin eine zur Oberfläche der Scheibe abgeschrägte Oberfläche aufweist und/oder der Kolben um die Kolbenachse drehfest in dem Adapter gelagert ist.

Durch die Abschrägung der Kolbenseite, die zur Scheibe hinweist, wird eine periodische lineare Bewegung des Kolbens als Stößel für eine Membranpumpe eines Lavage-Systems bei einer Drehung der Scheibe erreicht.

Ferner kann dabei vorgesehen sein, dass das der Scheibe gegenüberliegende Ende des Kolbens, das vorzugsweise abgerundet ausgebildet ist, bei angeschlossenem Lavage-System das Lavage-System antreibt, insbesondere bei angeschlossenem Lavage-System und sich drehender Scheibe der Kolben periodisch auf die Membran des Lavage-Systems stößt.

Die Abrundung des Kolbens soll eine Beschädigung der Membran durch Ecken und Kanten des Kolbens vermeiden.

Bevorzugt kann bei erfindungsgemäßen Adaptern vorgesehen sein, dass die Scheibe eine Exzenter ist, wobei bevorzugt der von der Welle entfernteste Bereich des Exzenters bei angeschlossenem Lavage-System das Lavage-System antreibt, insbesondere bei angeschlossenem Lavage-System der von der drehenden Welle entfernteste Bereich des sich drehenden Exzenters periodisch auf die Membran des Lavage-Systems stößt.

Die Aufgaben der Erfindung werden auch gelöst durch einen Adapter für einen Bohrantrieb zum Antreiben eines Lavage-Systems, umfassend eine erste Befestigungseinrichtung zum Anschließen des Adapters an den Bohrantrieb, eine zweite Befestigungseinrichtung zum Anschließen des Lavage-Systems an den Adapter und eine achsial drehbar gelagerte Welle, die an einem ersten Ende der Welle an den Bohrantrieb derart anschließbar ist, dass die Welle durch den Bohrantrieb drehbar ist, wobei die Welle eine Kurbelwelle umfasst, die einen Pleuel antreibt, wobei der Adapter über die zweite Befestigungseinrichtung derart an das Lavage-System anschließbar ist, dass der Pleuel das Lavage-System antreibt und wobei vorzugsweise der Pleuel periodisch auf eine Membran des Lavage-System stößt.

Die Verwendung einer Kurbelwelle führt zwar zu einer Unwucht, ist jedoch besonders einfach im Aufbau. Die Lagerung der Kurbelwelle muss stabiler sein, als bei der Verwendung ausgewuchteter Wellen und Scheiben.

Bei allen erfindungsgemäßen Adaptern kann vorgesehen sein, dass die erste Befestigungseinrichtung am ersten Ende der Welle realisiert ist, wobei vorzugsweise die erste Befestigungseinrichtung ein Schnellkupplungsstück für eine Schnellkupplung des Bohrantriebs ist und/oder die Welle zumindest eine Fläche, zumindest eine Ausnehmung und/oder zumindest einen Stift am ersten Ende der Welle aufweist, über den oder die über ein Gegenrastmittel des Bohrantriebs eine drehschlüssige Verbindung zu dem Bohrantrieb herstellbar oder hergestellt ist.

Da das Drehmoment des Bohrantriebs auf die Welle übertragen werden muss, ist eine erste Befestigungseinrichtung an der Welle ausreichend und stellt daher eine besonders einfache Ausführungsform der Erfindung dar.

Ferner kann für alle erfindungsgemäßen Adapter vorgesehen sein, dass die zweite Befestigungseinrichtung ein Bajonett-Verschluss ist, mit dem das Lavage-System mit dem Adapter verbindbar oder verbunden ist.

Gemäß einer weiteren Ausgestaltung der Erfindung kann vorgesehen sein, dass die Welle eine biegsame Welle ist oder eine Gelenkwelle, insbesondere eine Gleichlaufgelenkwelle, umfassend wenigstens ein Gelenk ist.

Hierdurch wird es ermöglicht, dass die lineare periodische Bewegung zum Antreiben der Membranpumpe nicht in Richtung der Drehachse des Bohrantriebs liegen muss. Dadurch sind anwenderfreundliche Lavage-Systeme mit Bohrantrieb aufbaubar.

Auch kann vorgesehen sein, dass der Adapter ein Gehäuse umfasst, das die Welle und vorzugsweise auch die Scheibe umschließt, wobei insbesondere der Adapter außer im Bereich der Befestigungseinrichtungen durch das Gehäuse vollständig abgeschlossen ist, so dass vorzugsweise der angeschlossene Adapter nach außen durch das Gehäuse abgeschlossen ist.

Durch das Gehäuse werden Fehlfunktionen vermieden und eine Gefahrenquelle durch schnell rotierende Bauteile gesichert.

Die Aufgaben der Erfindung werden auch gelöst durch einen Bohrantrieb umfassend einen solchen Adapter, bei dem die Welle des Adapters in einem Befestigungsmittel, insbesondere in einem Bohrfutter des Bohrantriebs befestigbar oder befestigt ist und die Welle durch den Bohrantrieb antreibbar oder angetrieben ist.

Dabei kann vorgesehen sein, dass der Bohrantrieb einen Elektromotor, einen Akkumulator oder eine Batterie, wenigstens eine Bedieneinrichtung und einen Griff umfasst, wobei der Akkumulator oder die Batterie an den Elektromotor angeschlossen ist und durch den Akkumulator oder die Batterie der Elektromotor mit elektrischer Energie speisbar ist, wobei der Elektromotor durch die Bedieneinrichtung steuerbar ist.

Die Aufgaben der Erfindung werden ferner gelöst durch ein Lavage-System umfassend einen solchen Adapter oder umfassend einen solchen Bohrantrieb bei dem das Lavage-System derart mit dem Adapter verbunden ist, dass die drehende Scheibe, vorzugsweise der Aufsatz, der Vorsprung oder der Exzenter, oder ein durch die drehende Scheibe oder eine Kurbelwelle periodisch linear bewegter Stößel, vorzugsweise der Pleuel oder der Kolben, das Lavage-System antreibt.

Dabei kann vorgesehen sein, dass das Lavage-System eine flexible Membran umfasst, auf die der Adapter bei drehender Welle mit einer periodischen Bewegung einwirkt, vorzugsweise durch periodische Stöße des linear bewegten Stößels.

Ferner kann vorgesehen sein, dass der periodische Antrieb durch den Adapter periodische Sprühstöße des Lavage-Systems bewirkt, wobei das Lavage-System an ein Flüssigkeitsreservoir anschließbar oder angeschlossen ist, wobei das Lavage-System mit dem Adapter eine Pumpeinrichtung des Lavage-Systems bildet, mit dem Flüssigkeit aus dem Flüssigkeitsreservoir in das Lavage-System pumpbar ist.

Der Erfindung liegt die überraschende Erkenntnis zugrunde, dass es mit einem einfach aufgebauten Adapter gelingt, in Operationssälen meist ohnehin vorhandene Bohrantriebe mit einfachen Lavage-Systemen zu kombinieren, um so einen möglichst kostengünstigen Aufbau eines Lavage-Systems mit Antrieb bereitzustellen. Dazu setzt der Adapter die Drehbewegung des Bohrantriebs in eine periodische Bewegung um, die auf die Membran eines vorhandenen Lavage-Systems einwirkt und so eine Membranpumpe bildet, die zur Erzeugung des periodischen Flüssigkeitsstahls verwendet wird. Dazu umfasst der Adapter erfindungsgemäß eine Befestigungseinrichtung zur Befestigung des Lavage-Systems am Adapter und eine Befestigungseinrichtung zur Befestigung des Bohrantriebs am Adapter. Der Adapter dient also als Zwischenstück für einen in Operationssälen üblicherweise vorhandenen Bohrantrieb und ein kostengünstigen Lavage-Vorsatz beziehungsweise ein Lavage-System.

Die verschiedenen Ausführungsformen der Erfindung stellen einfache Realisierungsvarianten des Erfindungsgedankens dar. Der Aufbau des Adapters bleibt in allen Fällen denkbar einfach. Zudem kann der Adapter mehrfach verwendet werden, während nur ein Lavage-Aufsatz oder ein Lavage-Vorsatz, vorliegend auch als Lavage-System bezeichnet, sterilisiert beziehungsweise ausgetauscht werden muss.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: eine schematische Querschnittansicht eines erfindungsgemäßen Lavage-Systems mit einem erfindungsgemäßen Adapter und einem Bohrantrieb;
- Figur 2:: eine schematische Querschnittansicht eines Adapters mit einem federnd gelagerten Kolben;
- Figur 3:: eine schematische Aufsicht auf einen Adapter mit einer Gelenkwelle und einem Pleuel bei geöffnetem Gehäuse;
- Figur 4:: eine schematische Aufsicht auf einen Adapter mit einem Exzenter und einem Pleuel;
- Figur 5:: eine schematische Querschnittansicht eines Adapters mit einer geneigten Scheibe und einem abgeschrägten Kolben; und
- Figur 6:: eine schematische Querschnittansicht eines Adapters mit einer Gelenkwelle und einem Pleuel mit Kugelgelenk.

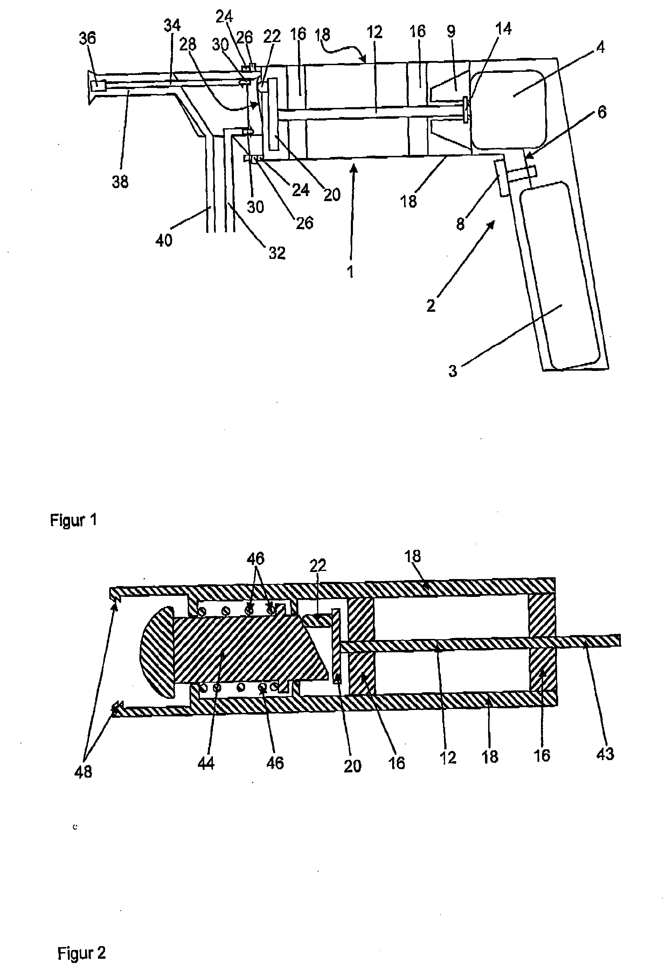

Figur 1 zeigt eine schematische Querschnittansicht eines erfindungsgemäßen Lavage-Systems mit Lavage-Aufsatz, Adapter 1 und Bohrantrieb 2. Der Bohrantrieb 2 umfasst einen Akkumulator 3 als Energiequelle und einen Elektromotor 4, der das Drehmoment für den Bohrantrieb 2 bereitstellt. Der Akkumulator 3 ist in einem Griff des Bohrantriebs 2 angeordnet. Der Elektromotor 4 ist über eine elektrische Leitung 6 mit dem Akkumulator 3 verbunden. Über eine Bedieneinrichtung 8 in Form eines Schalters 8 kann der Elektromotor 4 ein- und ausgeschaltet werden. Es ist auch denkbar, über die Bedieneinrichtung 8 die Drehzahl des Elektromotors 4 zu regeln.

Auf der Vorderseite des Bohrantriebs 2 befindet sich ein Bohrfutter 9 oder eine andere Befestigungseinrichtung. In diesem Bohrfutter 9 ist eine Welle 12 des Adapters 1 eingespannt. Die Welle 12 umfasst einen Stift 14, der ebenfalls zur drehschlüssigen Befestigung der Welle 12 mit dem Bohrantrieb 2 dient. Das erste Ende der Welle 12 mit dem Stift 14 ist im Sinne der vorliegenden Erfindung das erste Befestigungsmittel.

Zwei Halterungen 16 sorgen dafür, dass die Welle 12 positionsfest zu den Halterungen 16 aber drehbar in den Halterungen 16 gelagert ist. Die Halterungen 16 sind mit einem Gehäuse 18 verbunden, das den angeschlossenen Adapter 1 umschließt.

Am dem, dem ersten Ende der Welle 12 gegenüberliegenden Ende der Welle 12 ist eine Scheibe 20 in dem Adapter 1 angeordnet. Die Scheibe 20 ist kreisrund und die Achse der Welle 12 stimmt mit dem Zentrum der Kreisscheibe der Scheibe 20 überein. Auf der Scheibe 20 ist ein Aufsatz 22 exzentrisch angeordnet, so dass dieser sich bei drehender Scheibe 20 im Kreis bewegt.

An der Vorderseite des Adapters 1 ist ein Bajonett-Verschluss 24 angeordnet, der in kurze Stifte 26 des Lavage-Aufsatzes greift, so dass der Lavage-Aufsatz mit dem Adapter 1 lösbar verbunden ist. Das Lavage-System umfasst eine flexible Membran 28 auf die der Aufsatz 22 der Scheibe 20 drückt, wenn der Adapter 1, wie gezeigt, an den Lavage-Aufsatz angeschlossen ist. Der Aufsatz 22 drückt die Membran 28 also in der gezeigten Position ein. Die flexible Membran 28 kann beispielsweise aus Kautschuk gefertigt sein. Wenn die Scheibe 20 leicht gegen die Achse der Welle 12 geneigt ist, schlägt der Aufsatz 22 bei einer Drehung der Scheibe 20 periodisch auf die Membran 28.

Die Membran 28 bildet eine Wand eines Raums, der über Ventile 30 mit zwei Leitungen 32, 34 verbunden ist. Die erste Leitung 32 führt dem Raum eine Flüssigkeit zu, während die zweite Leitung 34 die Flüssigkeit zu einer Düse 36 führt, aus der die Flüssigkeit austreten kann.

Die mit Rückständen vermischte Flüssigkeit kann anschließend über eine Absaugleitung 38 wieder abgesaugt werden, die die zweite Leitung 34 koaxial umgibt. Anschließend kann die Flüssigkeit über eine Abführleitung 40 aus dem Lavage-System abgeführt werden.

Das Ventil 30 in der ersten Leitung 32 sperrt einen Flüssigkeitsaustritt aus dem Raum in die erste Leitung 32 hinein und erlaubt einen Flüssigkeitseintritt in den Raum hinein. Das Ventil 30 in der zweiten Leitung 34 sperrt einen Flüssigkeitseintritt in den Raum aus der zweiten Leitung 34 und erlaubt einen Flüssigkeitsaustritt aus dem Raum in die zweite Leitung 34 hinein. Der Raum mit der Membran 28 bildet so eine Membranpumpe, die durch den periodisch über die Membran 28 laufenden Aufsatz 22 angetrieben wird. Das Volumen des Raums wird verkleinert, wenn der Aufsatz 22 die Membran 28 eindrückt. Dann wird die Flüssigkeit aus dem Raum in die zweite Leitung 34 hinein gedrückt und als Sprühstoß aus der Düse 36 ausgestoßen. Wenn der Aufsatz 22 zu einer Position wandert, an der er nicht auf die Membran 28 drückt oder an der die Membran 28 weniger stark verformt wird, vergrößert sich das Volumen des Raums. Dadurch wird Flüssigkeit aus der ersten Leitung 32 durch das untere Ventil 30 in den Raum eingesaugt.

Bei fortwährend drehender Scheibe 20 beziehungsweise bei fortlaufend drehender Welle 12 und dadurch periodischem Eindrücken der Membran 28 durch den Aufsatz 22 erfolgen so periodische Sprühstöße der Flüssigkeit aus der Düse 36.

Alle Teile des Adapters 1 können aus Kunststoff bestehen und durch Spitzgießen gefertigt sein. Eine durch den Aufsatz 22 verursachte Unwucht der Scheibe 20 kann durch ein Gegengewicht (nicht gezeigt) auf der gegenüberliegenden Seite oder dem äußeren Rand der Scheibe 20 ausgeglichen werden.

In den Figuren 2 bis 6 sind jeweils nur noch Adapter gezeigt, die aber ohne weiteres an Lavage-Aufsätze und/oder Bohrantriebe, wie die in Figur 1 gezeigten, angeschlossen sein können, um Bohrantriebe oder Lavage-Systeme zu bilden.

Figur 2 zeigt eine schematische Querschnittansicht eines alternativen Adapters mit einer zentralen, drehbar gelagerten Welle 12. Ein erstes Ende 43 der Welle 12 dient als Befestigungsmittel zur Befestigung eines Bohrantriebs (nicht gezeigt). Dazu kann das erste Ende 43 der Welle 12 in den Bohrantrieb eingespannt werden.

Die Welle 12 ist drehbar in zwei Halterungen 16 gelagert. Die Halterungen 16 sind fest mit dem Gehäuse 18 des Adapters verbunden. Das Gehäuse 18 ist nach außen zylindrisch geformt. An dem, dem ersten Ende 43 gegenüberliegenden Ende der Welle 12 ist eine Scheibe 20 angeordnet, wobei die Scheibe 20 beliebig geformt sein kann, sofern sie sich innerhalb des Gehäuses 18 um die Achse der Welle 12 frei drehen lässt. Die Scheibe 20 könnte also auch durch eine abknickende Welle (nicht gezeigt) realisiert sein.

Auf der Scheibe 20 ist ein Aufsatz 22 angeordnet. Ebenso gut könnte die Scheibe 20 aber auch an der entsprechenden Stelle verformt sein oder die abknickende Welle hat einen weiteren Knick. Auch diese Ausführungsformen sind Ausgestaltungen des Adapters.

Der Aufsatz 22 drückt auf einen linear beweglichen Kolben 44, der im Inneren des Gehäuses 18 gelagert ist. Der Kolben 44 umfasst einen pilzförmigen Kopf, der zum periodischen Stoßen einer Membran eines mit dem Adapter gebildeten Lavage-Systems (nicht gezeigt) vorgesehen ist.

Der Kolben 44 wird mit einer Metallfeder 46 in Richtung der Scheibe 20 und damit gegen den Aufsatz 22 gedrückt. Der Kolben 44 hat einen rechteckigen Querschnitt und sitzt in rechteckigen Ausnehmungen in dem Gehäuse 18. Dadurch ist sichergestellt, dass der Kolben 44 nicht drehbar gelagert ist. Jede andere Geometrie des Kolbens 44 beziehungsweise der Ausnehmungen im Gehäuse 18, in denen der Kolben 44 gelagert ist, die eine Drehung des Kolbens 44 verhindern, erfüllen den gleichen Zweck.

Auf der der Scheibe 20 zugewandten Seite des Kolbens 44 ist der Kolben 44 abgeschrägt. Wenn sich die Welle 12 dreht, überstreicht der Aufsatz 22 das abgeschrägte Ende des Kolbens 44, wodurch der Kolben 44 periodisch ausgelenkt wird. Durch den Adapter wird auf diese Weise aus der Drehung der Welle 12 ein periodisches Stoßen des Kolbens 44 erzeugt.

An dem, dem ersten Ende 43 gegenüberliegenden Ende des Adapters ist auf der Innenseite des Gehäuses 18 eine Rastvorrichtung 48 angeordnet, die dazu ausgelegt ist, in eine Gegenrastvorrichtung eines Lavage-Aufsatzes (nicht gezeigt) zu greifen, so dass mit dem Lavage-Aufsatz und dem Adapter in einfacher Weise ein Lavage-System aufgebaut werden kann.

Alle Teile des Adapters bis auf die Feder 46 bestehen aus Kunststoff und werden durch Spitzgießen gefertigt. Grundsätzlich kann sogar die Feder 46 aus einem elastischen Kunstsoff gefertigt sein.

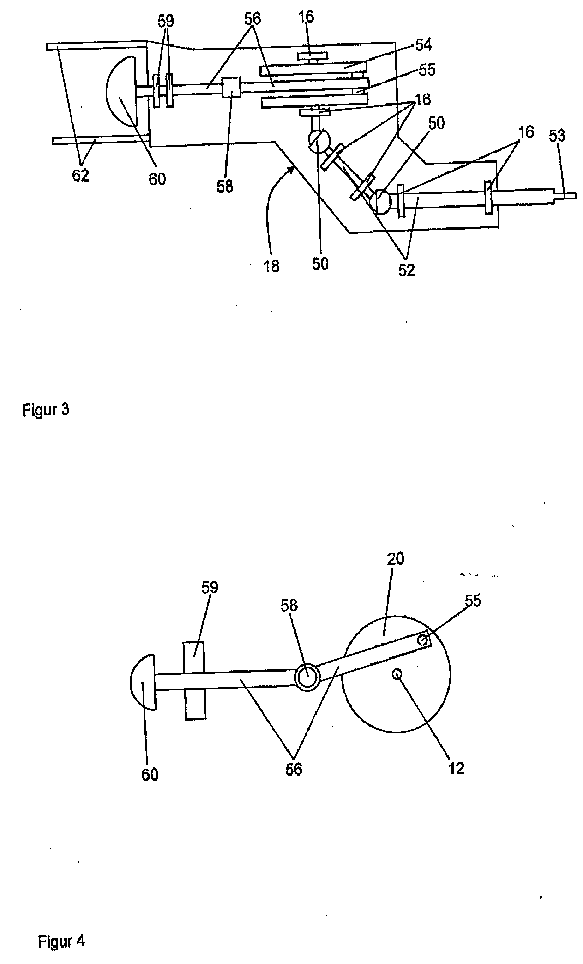

Figur 3 zeigt eine schematische Aufsicht auf einen weiteren Adapter bei geöffnetem Gehäuse 18. Dadurch ist der Aufbau im Inneren des Gehäuses 18 gut in Aufsicht zu erkennen. Der Adapter umfasst eine Gelenkwelle 52, deren Drehachse durch zwei Kugelgelenke 50 umgelenkt wird. An einem ersten Ende der Gelenkwelle 52 ist eine Abflachung 53 der Gelenkwelle 52 als erstes Befestigungsmittel vorgesehen, mit dem der Adapter mit einem Bohrantrieb (nicht gezeigt) verbunden werden kann.

Die Gelenkwelle 52 kann beispielsweise eine Gleichlaufgelenkachse sein oder eine einfach aufgebaute Gelenkwelle. Die Lage der Teile der Gelenkwelle 52 in dem Adapter ist durch Halterungen 16 definiert, die fest mit dem Gehäuse 18 verbunden sind.

Die Halterungen 16 definieren auch die Position einer Kurbelwelle 54, die drehbar mit der Gelenkwelle 52 verbunden ist. Die Kurbelwelle 54 ist über eine Drehachse 55 mit einem Pleuel 56 verbunden, der die Drehbewegung der Gelenkwelle 52 und der Kurbelwelle 54 in eine linear oszillierende Bewegung umwandelt. Dazu umfasst der Pleuel 56 ein Drehgelenk 58 und ist linear verschiebbar durch zwei Halterungen 59 gelagert. Während die Halterungen 16 der Gelenkwelle 52 und der Kurbelwelle 54 also eine Drehung in der Halterung 16 ermöglichen, sind die Halterungen 59 dazu ausgelegt, ein lineares Verschieben des Pleuels 56 durch die Halterungen 59 hindurch zu ermöglichen. Beide Halterungen 16, 59 sind also keine feste Verbindungen zu der Gelenkwelle 52 beziehungsweise zu dem Pleuel 56.

Anstatt einer Kurbelwelle 54 könnte auch eine Scheibe oder ein parallel angeordnetes Paar von Scheiben drehbar in dem Adapter gelagert und mit der Gelenkwelle 52 verbunden sein. Der Pleuel 56 ist dann exzentrisch an der Scheibe oder zwischen den Scheiben über eine Achse verbunden.

Der Pleuel 56 ist auf der Seite, an der ein Lavage-Aufsatz befestigt werden kann, durch einen abgerundeten Kopf 60 abgeschlossen. Im Bereich des Kopfs 60 ist das Gehäuse 18 verlängert und bildet dort eine zweite Befestigungseinrichtung 62 für ein Lavage-System (nicht gezeigt), das in die zweite Befestigungseinrichtung 62 eingesteckt werden kann.

Wenn ein Lavage-System mit Adapter durch Aufsetzen eines Lavage-Systems in die zweite Befestigungseinrichtung 62 und Befestigen eines Bohrantriebs an der Gelenkwelle 52 an dem Adapter aufgebaut wird, so wird bei angetriebener Gelenkwelle 52 der Pleuel 56 periodisch linear oszillierend bewegt, so dass der Kopf 60 periodisch auf eine Membran des Lavage-Systems stößt und dadurch eine Membranpumpe betrieben wird, die periodische Sprühstöße des Lavage-Systems bewirkt.

Die Gelenkwelle 52 dient ausschließlich dazu, dass die lineare Bewegung des Pleuels 56 und damit des Kopfs 60 in Richtung der Drehachse des Bohrantriebs erfolgt. Mit dem Lavage-System umfassend den Bohrantrieb und dem aufgesetzten Lavage-System kann dann nach Art einer Pistole gearbeitet werden. Alternativ dazu kann auch der Aufsatz, also das auf den Adapter aufzusetzende Lavage-System gebogen sein, so dass auf eine Gelenkwelle 52 im Adapter verzichtet werden kann und stattdessen eine normale Welle ohne Gelenke eingesetzt werden kann.

Figur 4 zeigt eine weitere alternative Ausführungsform eines Adapters in schematischer Aufsicht. Um den Adapter herum kann ein Gehäuse (nicht gezeigt) vorgesehen sein. Der Adapter umfasst eine Scheibe 20, die drehbar um eine Welle 12 gelagert ist. Die Welle 12 erstreckt sich auf der Rückseite der Scheibe 20 weiter (in Figur 4 in die Bildebene hinein) und endet an einer Befestigungseinrichtung für einen Bohrantrieb. Die Welle 12 bildet die Drehachse für die kreisförmige Scheibe 20.

Über eine Drehachse 55 ist ein Pleuel 56 exzentrisch mit der Scheibe 20 verbunden. Der Pleuel 56 umfasst ein Gelenk 58 und ist hinter dem Gelenk 58 durch eine Halterung 59 mit einer Durchführung für den von der Scheibe 20 abgewandten Gelenkarm des Pleuels 56 linear beweglich gehaltert. Der Pleuel 56 endet hinter der Halterung 59 mit einem Kopf 60, der als Kontakt für eine Membran dient.

Die Stoßrichtung des Pleuels 56 beziehungsweise des Kopfs 60 erfolgt hier senkrecht zur Drehachse der Welle 12, die hier als einfache Stange ausgebildet sein kann, und damit senkrecht zur Drehachse eines Bohrantriebs, wenn dieser an den Adapter angeschlossen ist. Die Halterung 59 für den Pleuel 56 und eine Halterung für die Scheibe 20, die sich hinter der Scheibe 20 befindet und in Figur 4 daher nicht zu sehen ist, sind fest miteinander verbunden. Die Scheibe 20 ist in der nicht zu sehenden Halterung drehbar gelagert. Mit den Halterungen 59 ist auch eine Befestigungseinrichtung (nicht gezeigt) zur Befestigung eines Lavage-Systems fest verbunden.

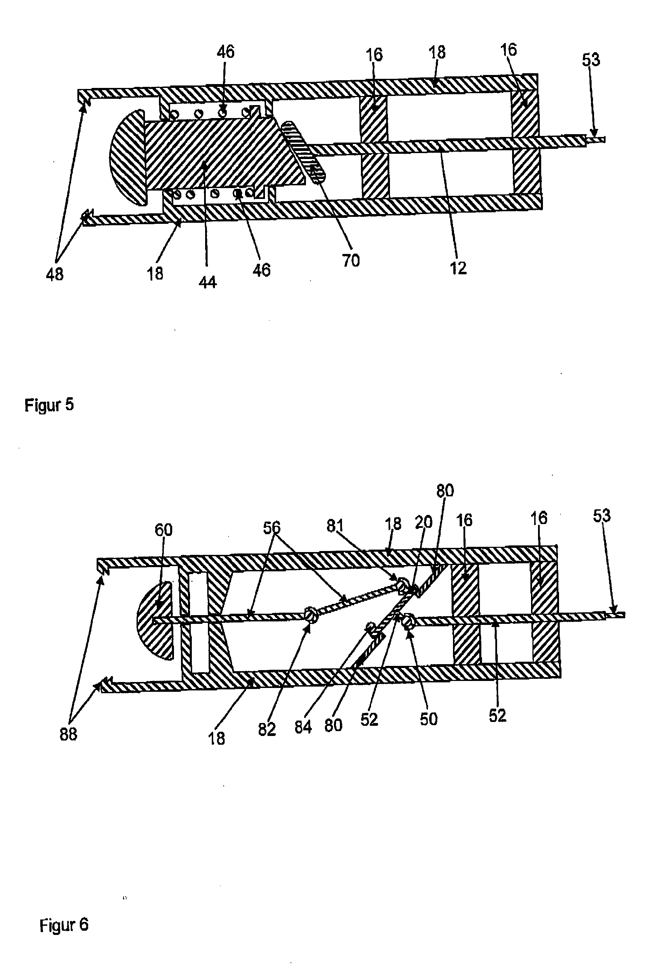

Figur 5 zeigt eine schematische Querschnittansicht einer weiteren alternativen Ausgestaltung eines Adapters für ein Lavage-System oder einen Bohrantrieb, das ähnlich dem nach Figur 2 aufgebaut ist. Anstatt einer Scheibe 20 mit einem Aufsatz 22 nach Figur 1 umfasst der vorliegende Adapter eine zur Welle 12 geneigte Scheibe 70 mit abgerundeter Kante. Die Scheibe 70 kann kreisrund, rotationssymmetrisch und im Symmetriezentrum mit der Welle 12 verbunden sein. Die Scheibe 70 kann aber auch eine Stange mit abgerundeten Enden sein, wobei die Stange ein eckiges oder auch ein rundes Profil haben kann.

Durch die Neigung der Scheibe 70 läuft die vordere Kante der Scheibe 70 über das abgeschrägte Ende eine Kolbens 44, der linear verschiebbar in einem Gehäuse 18 gelagert ist. Durch eine Feder 46 wird sichergestellt, dass der Kolben 44 auf die Scheibe 70 gedrückt wird, unabhängig von der Drehposition der Scheibe 70. Halterungen 16 halten die Welle 12 in Position.

Als Befestigungseinrichtung 53 für einen Bohrantrieb ist bei dem vorliegenden Adapter im Unterschied zu dem nach Figur 2 eine Abflachung 53 der aus dem Gehäuse 18 und der hinteren Halterung 16 (in Figur 5 rechts) herausragenden Welle 12 vorgesehen. Grundsätzlich könnte die Welle 12 auch durch einen Vierkant oder eine andere kantige Stange realisiert werden, die dann leicht in einer Befestigungseinrichtung des Bohrantriebs eingespannt werden kann.

An der Vorderseite des Adapters (in Figur 5 links) ist eine Rastvorrichtung 48 zur Befestigung eines Lavage-Systems (nicht gezeigt) angeordnet. Die Funktionsweise eines mit dem Adapter zusammengesetzten Lavage-Systems ist analog zu den bereits beschriebenen Ausführungsformen.

Anstatt einer geneigten Scheibe 70 könnte die Welle 12 auch einfach links der linken Halterung 16 abgewinkelt sein. Das linke Ende der Welle 12 würde dann ebenfalls kreisförmig über die abgeschrägte rechte Oberfläche des Kolbens 44 laufen und den gleichen Effekt erzielen, wie die vorliegende Scheibe 70. Nachteilig ist an einem solchen Aufbau allerdings die Unwucht, die durch die asymmetrische Welle 12 entsteht. Wenn die Welle 12 aus leichten Materialien aufgebaut ist oder keine großen Umdrehungsgeschwindigkeiten verwendet werden, wäre ein solcher Aufbau aber vorstellbar.

Figur 6 zeigt eine schematische Querschnittansicht eines weiteren Adapters. Der Adapter umfasst eine Gelenkwelle 52, die drehbar in Halterungen 16 gelagert ist. Am hinteren Ende (in Figur 6 rechts) ragt die Gelenkwelle 52 aus einem Gehäuse 18, das fest mit den Halterungen 16 verbunden ist, heraus und hat dort eine Abflachung 53 zur Befestigung eines Bohrantriebs. Über ein Gelenk 50 wird die Drehachse in der Gelenkwelle 52 umgelenkt. Das Gleiche ließe sich auch mit einer flexiblen Welle erreichen, die alternativ verwendet werden kann. Die umgelenkte Gelenkwelle 52 ist an einer Scheibe 20 befestigt, die über zwei Halterungen 80, die fest mit dem Gehäuse 18 verbunden sind, drehbar in dem Adapter gelagert ist.

Die Scheibe 20 umfasst ein exzentrisch befestigtes Kugelgelenk 81, das einen Pleuel 56 mit der Scheibe 20 verbindet. Der Pleuel 56 umfasst ein weiteres Kugelgelenk 82, das den Pleuel 56 in zwei Gelenkarme unterteilt, wobei der mit der Scheibe 20 verbundene Gelenkarm bei sich drehender Scheibe 20 eine Art Nutationsbewegung vollführt, die entlang einer vom Winkel abhängig verschobenen Kegeloberfläche verläuft, deren Kegelspitze durch das Kugelgelenk 82 gebildet wird und wobei sich der Schwerpunkt des Gelenkarms und des Kugelgelenks 82 bei einer Umdrehung sinusförmig in Richtung der Drehachse des Bohrantriebs verschiebt. Der von der Scheibe 20 abgewandte Gelenkarm des Pleuels 56 bewegt sich periodisch linear hin und her und treibt dabei einen Kopf 60 an, der an dem linken Ende des Pleuels 56 befestigt ist und der als Stoßkontakt zum Stoßen auf eine Membran einer Membranpumpe vorgesehen ist. Dazu kann ein Lavage-System über ein Innengewinde 88 am Gehäuse 18 mit dem Adapter verbunden werden. Der linke Gelenkarm des Pleuels 56 ist in zwei Halterungen 59 linear verschiebbar gelagert.

Um eine Unwucht des Systems auszugleichen ist auf der Scheibe 20 ein Gegengewicht 84 auf der dem Kugelgelenk 81 gegenüberliegenden Seite der Scheibe 20 befestigt. Die Funktionsweise eines mit dem Adapter aufgebauten Lavage-Systems ist analog den bisherigen Ausführungen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Adapter
- 2: Bohrantrieb
- 3: Akkumulator
- 4: Elektromotor
- 6: Elektrisch Leitung
- 8: Schalter / Betätigungseinrichtung
- 9: Bohrfutter
- 12: Welle
- 14, 26: Stift
- 16, 59, 80: Halterung
- 18: Gehäuse
- 20, 70: Scheibe
- 22: Aufsatz
- 24: Bajonett-Verschluss
- 28: Membran
- 30: Ventil
- 32, 34: Zuleitung
- 36: Düse
- 38, 40: Ableitung
- 43: Erstes Ende der Welle
- 44: Kolben
- 46: Feder
- 48: Rastvorrichtung
- 50, 81, 82: Kugelgelenk
- 52: Gelenkwelle
- 53: Abflachung
- 54: Kurbelwelle
- 55: Drehachse
- 56: Pleuel
- 58: Gelenk
- 60: Kopf
- 62: Befestigungseinrichtung
- 84: Gegengewicht
- 88: Innengewinde

## Patentansprüche

1. Adapter für einen Bohrantrieb zum Antreiben eines Lavage-Systems, umfassend
eine erste Befestigungseinrichtung (14, 53) zum Anschließen des Adapters (1) an den Bohrantrieb (2),
eine zweite Befestigungseinrichtung (24, 48, 62, 88) zum Anschließen des Lavage-Systems an den Adapter (1) und
eine achsial drehbar gelagerte Welle (12, 52), die an einem ersten Ende (43) der Welle (12, 52) an den Bohrantrieb (2) derart anschließbar ist, dass die Welle (12, 52) durch den Bohrantrieb (2) drehbar ist,
wobei der Adapter (1) eine Scheibe (20, 70) umfasst, die an einem zweiten Ende der Welle (12, 52) gegenüber dem ersten Ende (43) mit der Welle (12, 52) derart verbunden ist, dass sich bei einer Drehung der Welle (12, 52) auch die Scheibe (20, 70) dreht, wobei die Scheibe (20, 70) einen Aufsatz (22) oder einen Vorsprung auf der von der Welle (12, 52) abgewandten Seite umfasst, der exzentrisch angeordnet ist, und/oder die Scheibe (20, 70) zur Drehachse der Welle (12, 52) geneigt ist, wobei der Adapter (1) über die zweite Befestigungseinrichtung (24, 48, 62, 88) derart an das Lavage-System anschließbar ist, dass die drehende Scheibe (20, 70) auf der von der Welle (12, 52) abgewandten Seite oder über den Aufsatz (22) oder den Vorsprung das Lavage-System antreibt, **dadurch gekennzeichnet, dass** der Aufsatz (22) oder der Vorsprung oder die drehende Scheibe (20, 70) auf der von der Welle (12, 52) abgewandten Seite über einen Druckpunkt direkt auf eine Membran (28) des Lavage-Systems drückt, wobei beim Drehen der Scheibe (20, 70) der Druckpunkt periodisch über die Membran (28) läuft.

2. Adapter nach Anspruch 1, **dadurch gekennzeichnet, dass**
die Scheibe (20, 70) eine Exzenter ist.

3. Adapter nach Anspruch 2, **dadurch gekennzeichnet, dass**
der von der Welle (12, 52) entfernteste Bereich des Exzenters bei angeschlossenem Lavage-System das Lavage-System antreibt.

4. Adapter nach Anspruch 3, **dadurch gekennzeichnet, dass**
bei angeschlossenem Lavage-System der von der drehenden Welle (12, 52) entfernteste Bereich des sich drehenden Exzenters periodisch auf die Membran (28) des Lavage-Systems stößt.

5. Adapter nach einem der vorangehenden Ansprüche, **dadurch gekennzeichnet, dass**
die erste Befestigungseinrichtung (14, 53) am ersten Ende (43) der Welle (12, 52) realisiert ist.

6. Adapter nach Anspruch 5, **dadurch gekennzeichnet, dass**
die erste Befestigungseinrichtung (14, 53) ein Schnellkupplungsstück für eine Schnellkupplung des Bohrantriebs (2) ist und/oder die Welle (12, 52) zumindest eine Fläche (52), zumindest eine Ausnehmung und/oder zumindest einen Stift (12) am ersten Ende (43) der Welle (12, 52) aufweist, über den oder die über ein Gegenrastmittel des Bohrantriebs (2) eine drehschlüssige Verbindung zu dem Bohrantrieb (2) herstellbar oder hergestellt ist.

7. Adapter nach einem der vorangehenden Ansprüche, **dadurch gekennzeichnet, dass**
die zweite Befestigungseinrichtung (24, 48, 62, 88) ein Bajonett-Verschluss (24) ist, mit dem das Lavage-System mit dem Adapter (1) verbindbar oder verbunden ist.

8. Adapter nach einem der vorangehenden Ansprüche, **dadurch gekennzeichnet, dass**
die Welle (12, 52) eine biegsame Welle ist oder eine Gelenkwelle (52) umfassend wenigstens ein Gelenk (50) ist.

9. Adapter nach einem der vorangehenden Ansprüche, **dadurch gekennzeichnet, dass**
der Adapter (1) ein Gehäuse (18) umfasst, das die Welle (12, 52) und die Scheibe (20, 70) umschließt.

10. Adapter nach Anspruch 9, **dadurch gekennzeichnet, dass**
der Adapter (1) außer im Bereich der Befestigungseinrichtungen (14, 24, 26, 48, 53, 62, 88) durch das Gehäuse (18) vollständig abgeschlossen ist, so dass der angeschlossene Adapter (1) nach außen durch das Gehäuse (18) abgeschlossen ist.

11. Bohrantrieb umfassend einen Adapter nach einem der Ansprüche 1 bis 10, **dadurch gekennzeichnet, dass**
die Welle (12, 52) des Adapters (1) in einem Befestigungsmittel (9) des Bohrantriebs (2) befestigbar oder befestigt ist und die Welle (12, 52) durch den Bohrantrieb (2) antreibbar oder angetrieben ist.

12. Bohrantrieb nach Anspruch 11, **dadurch gekennzeichnet, dass**
der Bohrantrieb (2) einen Elektromotor (4), einen Akkumulator (3) oder eine Batterie, wenigstens eine Bedieneinrichtung (8) und einen Griff umfasst, wobei der Akkumulator (3) oder die Batterie an den Elektromotor (4) angeschlossen ist und durch den Akkumulator (3) oder die Batterie der Elektromotor (4) mit elektrischer Energie speisbar ist, wobei der Elektromotor (4) durch die Bedieneinrichtung (8) steuerbar ist.

13. Lavage-System umfassend einen Adapter nach einem der Ansprüche 1 bis 10 oder umfassend einen Bohrantrieb nach Anspruch 11 oder 12, **dadurch gekennzeichnet, dass**
das Lavage-System derart mit dem Adapter (1) verbunden ist, dass die drehende Scheibe (20, 70), der Aufsatz (22), der Vorsprung oder der Exzenter das Lavage-System antreibt.

14. Lavage-System nach Anspruch 13, **dadurch gekennzeichnet, dass**
das Lavage-System eine flexible Membran (28) umfasst, auf die der Adapter (1) bei drehender Welle (12, 52) mit einer periodischen Bewegung einwirkt.

15. Lavage-System nach einem der Ansprüche 13 oder 14, **dadurch gekennzeichnet, dass**
der periodische Antrieb durch den Adapter (1) periodische Sprühstöße des Lavage-Systems bewirkt, wobei das Lavage-System an ein Flüssigkeitsreservoir anschließbar oder angeschlossen ist, wobei das Lavage-System mit dem Adapter (1) eine Pumpeinrichtung des Lavage-Systems bildet, mit dem Flüssigkeit aus dem Flüssigkeitsreservoir in das Lavage-System pumpbar ist.

## Claims

1. An adapter for a drill drive for driving a lavage system, comprising:
a first attachment device (14, 53) for connecting the adapter (1) to the drill drive (2);
a second attachment device (24, 48, 62, 88) for connecting the lavage system to the adapter (1); and
a shaft (12, 52), which is mounted so as to be axially rotatable and which can be connected at a first end (43) of the shaft (12, 52) to the drill drive (2) in such a way that the shaft (12, 52) can be rotated by the drill drive (2);
the adapter (1) comprising a disk (20, 70) connected to the shaft (12, 52) at a second end of the shaft (12, 52) opposite the first end (43) in such way that the disk (20, 70) also rotates during a rotation of the shaft (12, 52), the disk (20, 70) on the side facing away from the shaft (12, 52) comprising an attachment (22) or a projection, which is disposed eccentrically, and/or the disk (20, 70) being inclined toward the rotational axis of the shaft (12, 52), the adapter (1) being connectable to the lavage system by way of the second attachment device (24, 48, 62, 88) in such way that the rotating disk (20, 70) drives the lavage system on the side facing away from the shaft (12, 52) or by way of the attachment (22) or the projection, **characterized in that** the attachment (22) or the projection or the rotating disk (20, 70) on the side facing away from the shaft (12, 52) presses directly onto a diaphragm (28) of the lavage system by way of a pressure point, wherein the pressure point periodically runs across the diaphragm (28) during the rotation of the disk (20, 70).

2. The adapter according to claim 1, **characterized in that**
the disk (20, 70) is an eccentric disk.

3. The adapter according to claim 2, **characterized in that**
the region of the eccentric disk lying furthest from the shaft (12, 52) drives the lavage system when the lavage system is connected.

4. The adapter according to claim 3, **characterized in that**
the region of the rotating eccentric disk lying furthest from the rotating shaft (12, 52) periodically impinges on the diaphragm (28) of the lavage system when the lavage system is connected.

5. An adapter according to any one of the preceding claims, **characterized in that** the first attachment device (14, 53) is implemented at the first end (43) of the shaft (12, 52).

6. The adapter according to claim 5, **characterized in that**
the first attachment device (14, 53) is a quick-action coupling piece for a quick-action coupling of the drill drive (2) and/or the shaft (12, 52) has at least one surface (52), at least one recess and/or at least one pin (12) at the first end (43) of the shaft (12, 52), by way of which a rotationally locked connection can be or is established to the drill drive (2) via a counter detent means of the drill drive (2).

7. An adapter according to any one of the preceding claims, **characterized in that** the second attachment device (24, 48, 62, 88) is a bayonet catch (24), by way of which the lavage system can be or is connected to the adapter (1).

8. An adapter according to any one of the preceding claims, **characterized in that** the shaft (12, 52) is a flexible shaft or a propeller shaft (52) comprising at least one joint (50).

9. An adapter according to any one of the preceding claims, **characterized in that** the adapter (1) comprises a housing (18) that encloses the shaft (12, 52) and the disk (20, 70).

10. The adapter according to claim 9, **characterized in that**
the adapter (1) is entirely sealed by the housing (18), except in the region of the attachment devices (14, 24, 26, 48, 53, 62, 88), so that the connected adapter (1) is sealed by the housing (18) with respect to the outside.

11. A drill drive comprising an adapter according to any one of claims 1 to 10, **characterized in that**
the shaft (12, 52) of the adapter (1) can be or is attached in an attachment means (9) of the drill drive (2) and the shaft (12, 52) can be or is driven by the drill drive (2).

12. The drill drive according to claim 11, **characterized in that**
the drill drive (2) comprises an electric motor (4), a rechargeable battery (3) or a battery, at least one operating device (8) and a handle, wherein the rechargeable battery (3) or the battery is connected to the electric motor (4) and the electric motor (4) can be supplied with electrical energy by the rechargeable battery (3) or the battery, wherein the electric motor (4) can be controlled by the operating device (8).

13. A lavage system comprising an adapter according to any one of claims 1 to 10, or comprising a drill drive according to claim 11 or 12, **characterized in that** the lavage system is connected to the adapter (1) in such a way that the rotating disk (20, 70), the attachment (22), the projection or the eccentric disk drives the lavage system.

14. The lavage system according to claim 13, **characterized in that**
the lavage system comprises a flexible diaphragm (28) on which the adapter (1) acts with a periodic movement when the shaft (12, 52) rotates.

15. The lavage system according to either claim 13 or 14, **characterized in that**
the periodic driving by the adapter (1) brings about periodic spray squirts of the lavage system, wherein the lavage system can be or is connected to a liquid reservoir, wherein the lavage system, together with the adapter (1), forms a pumping device of the lavage system, by way of which liquid can be pumped from the liquid reservoir into the lavage system.

## Revendications

1. Adaptateur pour un entraînement de forage destiné à entraîner un système de lavage, comprenant
un premier dispositif de fixation (14, 53) pour le raccordement de l'adaptateur (1) à l'entraînement de forage (2),
un deuxième dispositif de fixation (24, 48, 62, 88) pour le raccordement du système de lavage à l'adaptateur (1), et
un arbre (12, 52) monté de façon à pouvoir tourner autour d'un axe, lequel peut être raccordé à l'entraînement de forage (2) par une première extrémité (43) de l'arbre (12, 52), de sorte que l'entraînement de forage (2) fait tourner l'arbre (12, 52),
dans lequel l'adaptateur (1) comprend un disque (20, 70) relié de telle façon à l'arbre (12, 52) à une deuxième extrémité de l'arbre (12, 52) opposée à la première extrémité (43), que le disque (20, 70) tourne également pendant une rotation de l'arbre (12, 52), dans lequel le disque (20, 70) comprend une coiffe (22) ou une saillie du côté détourné de l'arbre (12, 52), laquelle est agencée de façon excentrée, et/ou le disque (20, 70) est incliné vers l'axe de rotation de l'arbre (12, 52), dans lequel l'adaptateur (1) peut être raccordé de telle façon au système de lavage par le biais du deuxième dispositif de fixation (24, 48, 62, 88), que le disque rotatif (20, 70) entraîne le système de lavage du côté détourné de l'arbre (12, 52) ou par le biais de la coiffe (22) ou de la saillie, **caractérisé en ce que** la coiffe (22) ou la saillie ou le disque rotatif (20, 70) appuie directement par un point d'appui sur une membrane (28) du système de lavage du côté détourné de l'arbre (12, 52), le point d'appui se déplaçant périodiquement sur la membrane (28) pendant la rotation du disque (20, 70).

2. Adaptateur selon la revendication 1, **caractérisé en ce que** le disque (20, 70) est un disque excentrique.

3. Adaptateur selon la revendication 2, **caractérisé en ce que**
la région du disque excentrique la plus éloignée de l'arbre (12, 52) entraîne le système de lavage lorsque le système de lavage est raccordé.

4. Adaptateur selon la revendication 3, **caractérisé en ce que** lorsque le système de lavage est raccordé, la région du disque excentrique en rotation la plus éloignée de l'arbre (12, 52) en rotation bute contre la membrane (28) du système de lavage.

5. Adaptateur selon l'une des revendications précédentes, **caractérisé en ce que**
le premier dispositif de fixation (14, 53) est réalisé à la première extrémité (43) de l'arbre (12, 52).

6. Adaptateur selon la revendication 5, **caractérisé en ce que** le premier dispositif de fixation (14, 53) est une pièce d'accouplement rapide pour un accouplement rapide de l'entraînement de forage (2) et/ou **en ce que** l'arbre (12, 52) comporte au moins une surface (52), au moins un évidement et/ou au moins une tige (12) à la première extrémité (43) de l'arbre (12, 52), permettant de réaliser une liaison solidaire en rotation avec l'entraînement de forage (2), ou réalisant celle-ci par le biais d'un moyen de contre-encliquetage de l'entraînement de forage (2).

7. Adaptateur selon l'une des revendications précédentes, **caractérisé en ce que**
le deuxième dispositif de fixation (24, 48, 62, 88) est une fermeture à baïonnette (24), avec laquelle le système de lavage peut être relié ou est relié à l'adaptateur (1).

8. Adaptateur selon l'une des revendications précédentes, **caractérisé en ce que**
l'arbre (12, 52) est un arbre flexible ou un arbre articulé (52) comprenant au moins une articulation (50).

9. Adaptateur selon l'une des revendications précédentes, **caractérisé en ce que**
l'adaptateur (1) comprend un boîtier (18) renfermant l'arbre (12, 52) et le disque (20, 70).

10. Adaptateur selon la revendication 9, **caractérisé en ce que**
l'adaptateur (1) est entièrement enfermé dans le boîtier (18) hormis dans la région des dispositifs de fixation (14, 24, 26, 48, 53, 62, 88), de sorte que l'adaptateur (1) enfermé est isolé de l'extérieur par le boîtier (18).

11. Entraînement de forage comprenant un adaptateur selon l'une des revendications 1 à 10, **caractérisé en ce que**
l'arbre (12, 52) de l'adaptateur (1) peut être fixé ou est fixé dans un moyen de fixation (9) de l'entraînement de forage (2), et l'arbre (12, 52) peut être entraîné ou est entraîné par l'entraînement de forage (2).

12. Entraînement de forage selon la revendication 11, **caractérisé en ce que** l'entraînement de forage (2) comprend un moteur électrique (4), un accumulateur (3) ou une batterie, au moins un dispositif de commande (8) et un manche, dans lequel l'accumulateur (3) ou la batterie est raccordé(e) au moteur électrique (4), et le moteur électrique (4) peut être alimenté en énergie électrique par l'accumulateur (3) ou la batterie, le moteur électrique (4) pouvant être commandé par le dispositif de commande (8).

13. Système de lavage comprenant un adaptateur selon l'une des revendications 1 à 10 ou comprenant un entraînement de forage selon la revendication 11 ou 12, **caractérisé en ce que**
le système de lavage est relié à l'adaptateur (1) de manière à ce que le disque rotatif (20, 70), la coiffe (22), la saillie ou le disque excentrique entraîne le système de lavage.

14. Système de lavage selon la revendication 13, **caractérisé en ce que** le système de lavage comprend une membrane souple (28) sur laquelle agit l'adaptateur (1) avec un mouvement périodique pendant la rotation du disque (12, 52).

15. Système de lavage selon l'une des revendications 13 ou 14, **caractérisé en ce que**
l'entraînement périodique par l'adaptateur (1) provoque des jets périodiques du système de lavage, dans lequel le système de lavage pouvant être ou étant raccordé à un réservoir de liquide, dans lequel le système de lavage forme un dispositif de pompage du système de lavage avec l'adaptateur (1), permettant de pomper le liquide hors du réservoir de liquide et dans le système de lavage.
